# EUROPEAN PATENT APPLICATION

(11) **EP 1 741 435 A1**
(43) Date of publication of application: **10.01.2007**
(21) Application number: 04730184.1
(22) Date of filing: 29.04.2004
(51) Int. Cl.: A61K 31/64, A61K 31/155, A61K 31/426, A61K 9/22, A61P 3/10

(54) **ORAL MODIFIED-RELEASE LOZENGES AND THEIR PREPARATION METHOD**

(71) Applicant: Lotus Pharmaceutical Co., Ltd., Da-an Chiu, Taipei City 106 (TW)
(72) Inventor: WANG, Kuenjen, Taipei,Taiwan 106 (CN); LIN, Tongho, Taipei,Taiwan 106 (CN)
(74) Representative: Schoppe, Fritz
(86) International application number: PCT/CN2004/000419
(87) International publication number: WO 2005/105109

(57) **Abstract**

The present invention is related to a composition for a modified-release oral tablet, **characterized in that** comprising of:
a micronized active ingredient for lowering blood glucose level with a particle size distribution in the range of:
(1) at least 50% of the particles having a size ≤ 5 microns;
(2) at least 90% of the particles having a size ≤15 microns; and
(3) at least 95% of the particles having a size ≤25 microns; and

a modified-release agent for controlling the release of the active ingredient; and a method for preparation of the composition.

## Description

### FIELD OF THE INVENTION

The present invention is related to a composition for a modified-release oral tablet, especially related to a composition comprising of a pharmaceutically effective amount of micronized active ingredient for lowering blood glucose level and a modified-release agent comprising of hydrophilic polymers, and a method for preparation of the novel composition for a modified-release oral tablet.

### BACKGROUND OF THE INVENTION

Diabetes mellitus is a chronic disease with a hyperglycemia symptom resulted by a wide variety of causes, always concomitant with a retinopathy, glomerulosclerosis, and some kidney, neural or vascular lesions or complications, and has become the fifth leading cause of death in Taiwan, from 0.37 persons at the year of 1960 booming to 4.1 persons at the year 1999 per ten thousands of people. Diabetes is often categorized into type I and type II, type I diabetes is an insulin-dependent diabetes mellitus (IDDM), at early time it is also called "juvenile diabetes", the patient of IDDM is easily incurred by an acute ketoacidosis when insulin is not injected; type II diabetes is a non insulin-dependent diabetes mellitus(NIDDM), also is called adult diabetes, the onset of NIDDM is mainly related to the abnormal function of metabolism, such as insulin-resistance and functional defect of P cell, and hence the patients of NIDDM have to be treated with an oral drug for lowering blood glucose level.

Generally speaking, orally administered drugs for lowering blood glucose level can be divided into four categories: sulfonylureas, biguanides, thiazolidinedione, and α-glucosidase inhibitor. Sulfonylureas is a kind of orally administered drug major for use in the treatment of adult diabetes for lowering blood glucose, prescribed mainly for a patient who is not overweight and has β cells that are still functionable. The mechanism of sulfonylureas is to stimulate the secretion of insulin by pancreas, and to make insulin-containing granules in β cells undergo degranulation, therefore, insulin is released. In addition, the metabolism of insulin in liver can be reduced and make the number of receptors for insulin to increase. The side effects of sulfonylureas are rare, mostly are among inhibition of iodine ion entry into the thyroid gland, hypoglycemia by over-dosage, nauseate, emetic, or itching. Secondary generation of sulfonylurea, such as Gliclazide, Glipizide and Glibenclamide, is often prescribed in hospitals of Taiwan; biguanides is less dangerous for induction of hypoglycemia than sulfonylureas and hence is the best medication for overweight patients; the therapeutic mechanism of thiazolidinedione is to reduce the resistance to insulin in muscles and adipose tissues, therefore to lower the synthesis of endogenous glucose; α-glucosidase inhibitor is to inhibit the absorption of amylogen and disaccharide by inhibition of α-glucosidase in villi around the small intestine, and hence the increase of blood glucose level after a meal is lowered, meanwhile, this drug is often prescribed in combination with other oral drugs for lowering blood glucose level.

In aspect of a disease management, it is very important to use a modified-release drug for lowering blood glucose level to diabetes patients for achieving a steady and sequential therapeutic effect. In particular, a diabetes patient always has to take a drug in a long term for controlling the disease condition and hence a medication that can bring the best therapeutic efficiency should be developed for whom has to keep taking the drug, and the side effect as caused by the drug administration can be simultaneously avoided. Duration of drug efficiency is achieved by the modified-release drug because the maintenance for an effective amount of the drug in blood is prolonged, resulting in advantages that the frequency for administration can be lowered and the release rate and profile are more similar to a physical and chronic treatment; also, higher dosage is not necessary as a means for prolonging the dug efficiency, and the side effect possibly generated or occurrence of resistance is therefore reduced. For instances, micronized Gliclazide can be administered by a dosage of 80 mg two times per day, however, the non-micronized active ingredient can not achieve a clinically effective treatment efficiency by the same course due to the incomplete release of the drug by poor solubility. U.S. Patent Nos. 6,451,339 and 6,537,578 are related to a preparation of agent aiming for the object of controlled-release drug. The present invention is to invent a modified-release oral tablet in accordance with the above described object by means of controlling drug solubility for diabetes patients, and better safety and therapeutic efficiency for the drug can be achieved.

### SUMMARY OF THE INVENTION

The object of the present invention is related to a composition for a modified-release oral tablet, characterized in that comprising of a micronized active ingredient for lowering blood glucose level and a modified-release agent for controlling of the release of the active ingredient.

By a dissolution test, the composition as prepared by a micronized active ingredient for lowering blood glucose level and a modified-release agent has a better dissolution rate than the one comprising of a non-micronized active ingredient for lowering blood glucose level with a lapse of time.

The other object of the present invention is related to a method for preparation of above described composition for a modified-release oral tablet, first an active ingredient for lowering blood glucose level is micronized to a particle size distribution within the range of: (1) at least 50% of the particles having a size ≤ 5 microns; (2) at least 90% of the particles having a size ≤15 microns; and (3) at least 95% of the particles having a size ≤25 microns; then the said micronized active ingredient for lowering blood glucose level is mixed with a modified-release agent, subject to granulation, then dried and trimmed; and the resulting granulate is tabletted by the addition of an excipient, and a tablet with a hardness from 4 to 8 Kg is prepared.

The further object of the present invention is to provide a micronized composition for a modified-release oral tablet and a method for preparation of the composition, the composition as prepared by the present method possesses a better stability for absorption.

Another object of the present invention is to micronize the main ingredient via a micronization technique, and hence a lower dosage for the main ingredient is sufficient to achieve an efficiency similar to the one as achieved by the present therapeutic dosage for clinically application.

### BRIEF DESCRIPTION OF THE DRAWINGS

Fig. 1 is a dissolution profile for an oral tablet composition of the present invention which comprises of a micronized active ingredient Gliclazide for lowering blood glucose level with a lapse of time in purified water.

Fig. 2 is a dissolution profile comparison between an oral tablet composition comprising of a micronized active ingredient Gliclazide for lowering the blood glucose level (diamond shape) and an oral tablet composition comprising of a non-micronzied active ingredient for lowering the blood glucose level(rectangular shape) with a lapse of time in purified water.

Fig. 3 is a concentration change profile of the present composition in blood with a lapse of time.

### DETAILED DESCRIPTION OF THE PREFERRED EMBODIMENT

The present invention is related to a novel composition for a modified-release oral tablet, the ingredients mainly contained therein are a micronized active ingredient for lowering blood glucose level and a modified-release agent comprising of hydrophilic polymers, achieving a modified-release effect for the active ingredient for lowering blood glucose level.

The active ingredient for lowering blood glucose can be any active ingredients as used in any drugs for lowering blood glucose level, preferably sulfonylureas is used as the active ingredient of the present invention for lowering blood glucose level, if necessary, two or more active ingredients can be combined to achieve a better therapeutic efficiency, by selection from Gliclazide, Glipizide, Glibenclamide, Gliquidonem, Glimepiride and the combination thereof, etc.. The amount of active ingredient in the present composition, Gliclazide as an example, is preferably 30-60mg. The active ingredient is characterized by undergoing micronization, and preferably with a particle size distribution in the range of:

(1) at least 50% of the particles having a size ≤ 5 microns;

(2) at least 90% of the particles having a size ≤15 microns; and

(3) at least 95% of the particles having a size ≤25 microns

The modified-release agent as used in the present invention is preferably selected from the following substances: hydroxypropylmethyl cellulose, methyl cellulose, polyvinylpyrrolidone, hydroxylpropyl cellulose, vinylacetate copolymers, polyethylene oxides and the combination thereof, and the viscosity is preferably from 4,000 cps to 100,000cps, therefore the persons skilled in this art can adjust the components in the modified-release agent according to the chosen active ingredient for lowering blood glucose level for optimization of the modified-release efficiency. The amount of the modified-release agent is about 10-40% by weight of the tablet composition.

Many pharmaceutically acceptable and inert excipients, such as a bulking agent, a diluent, a lubricant, a correctant, a disintegrating agent, a binding agent, a coloring agent, or a sweetener can be added into the composition at optimal instance during the preparation process of the present tablet; sucrose, TiO2, microcrystalline, sorbitol, magnesium stearate or talc can also be added before the tabletting step or be added at the time of mixing of the active ingredient for lowering blood glucose level and modified-release agent. The hardness of the tablet after a tabletting step is preferably about 4-8Kg.

The suggested frequency for administration of the tablet of the present invention is one time per day and one to two tablets per time, and application use for the tablet of the present invention is not limited to diabetes treatment.

### Example 1

An oral tablet composition comprising of the drug Gliclazide for lowering blood glucose level is prepared as follows:

| | |
|---|---|
| Micronized Gliclazide | 30 mg |
| Lactose | 30 mg |
| SiO₂ | 1.6 mg |
| Hydroxypropylmethylcellulose | 84 mg |
| Polyvinylpyrrolidone | 8 mg |
| Polysorbate 80 | 3.2 mg |
| Magnesium stearate | 1.6 mg |
| Talc | 1.6 mg |
| Total | 160 mg |

The ingredients in the above composition are subject to the processes as described below to obtain a tablet of the present invention:

(1)mixing of micronized Glicazide, lactose, SiO2, hydroxypropylmethylcellulose and polyvinylpyrrolidone;

(2)dissolving polysorbate 80 in an aqueous solution of ethanol;

(3)pouring the solution of (2) into the mixed powder of (1), and the resulting wet agglomeration subject to granulation, and then dried and milled(#20 mesh);

(4)further mixing the granulate as obtained from (3) with magnesium stearate and talc;

(5) tabletting a tablet with a hardness from 4 to 8 Kg by a rotary tabletting machine.

### Example 2

An oral tablet composition comprising of the drug Gliclazide for lowering blood glucose level is prepared as follows:

| | |
|---|---|
| Micronized Gliclazide | 60 mg |
| Lactose | 60 mg |
| SiO₂ | 3.2 mg |
| Hydroxypropylmethylcellulose | 168 mg |
| Polyvinylpyrrolidone | 16 mg |
| Polysorbate 80 | 6.4 mg |
| Magnesium stearate | 3.2 mg |
| Talc | 3.2 mg |
| Total | 320 mg |

### Example 3

An oral tablet composition comprising of the drug Gliclazide for lowering blood glucose level is prepared as follows:

| | |
|---|---|
| Micronized Gliclazide | 30 mg |
| Mannitol | 90 mg |
| SiO₂ | 1.6 mg |
| Methylcellulose | 25.6 mg |
| Polyvinylpyrrolidone | 6.4 mg |
| Sodium Lauryl Sulfate | 3.2 mg |
| Magnesium stearate | 1.6 mg |
| Talc | 1.6 mg |
| Total | 160 mg |

### Example 4

An oral tablet composition comprising of the drug Gliclazide for lowering blood glucose level is prepared as follows:

| | |
|---|---|
| Micronized Gliclazide | 60 mg |
| Mannitol | 180 mg |
| SiO₂ | 3.2 mg |
| Methylcellulose | 51.2 mg |
| Polyvinylpyrrolidone | 12.8 mg |
| Sodium Lauryl Sulfate | 6.4 mg |
| Magnesium stearate | 3.2 mg |
| Talc | 3.2 mg |
| Total | 320 mg |

### Example 5

An oral tablet composition comprising of the drug Gliclazide for lowering blood glucose level is prepared as follows:

| | |
|---|---|
| Micronized Gliclazide | 30 mg |
| Microcrystalline cellulose | 60 mg |
| Lactose | 30 mg |
| SiO₂ | 1.6 mg |
| Hydroxypropylmethyl cellulose | 24 mg |
| Polyvinylpyrrolidone | 4.8 mg |
| Polysorbate 80 | 6.4 mg |
| Magnesium stearate | 1.6 mg |
| Talc | 1.6 mg |
| Total | 160 mg |

### Example 6

An oral tablet composition comprising of the drug Gliclazide for lowering blood glucose level is prepared as follows:

| | |
|---|---|
| Micronized Gliclazide | 60 mg |
| Microcrystalline cellulose | 120 mg |
| Lactose | 60 mg |
| SiO₂ | 3.2 mg |
| Hydroxypropylmethyl cellulose | 48 mg |
| Polyvinylpyrrolidone | 9.6 mg |
| Polysorbate 80 | 12.8 mg |
| Magnesium stearate | 3.2 mg |
| Talc | 3.2 mg |
| Total | 320 mg |

### Example 7

An oral tablet composition comprising of the drug Gliclazide for lowering blood glucose level is prepared as follows:

| | |
|---|---|
| Micronized Gliclazide | 30 mg |
| Lactose | 90 mg |
| SiO₂ | 1.6 mg |
| Hydroxypropylmethyl cellulose | 24 mg |
| Polyvinylpyrrolidone | 8 mg |
| Polysorbate 80 | 3.2 mg |
| Magnesium stearate | 1.6 mg |
| Talc | 1.6 mg |
| Total | 160 mg |

### Example 8

An oral tablet composition comprising of the drug Gliclazide for lowering blood glucose level is prepared as follows:

| | |
|---|---|
| Micronized Gliclazide | 60 mg |
| Lactose | 180 mg |
| SiO₂ | 3.2 mg |
| Hydroxypropylmethyl cellulose | 48 mg |
| Polyvinylpyrrolidone | 16 mg |
| Polysorbate 80 | 6.4 mg |
| Magnesium stearate | 3.2 mg |
| Talc | 3.2 mg |
| Total | 320 mg |

Dissolution Test

-in vitro testing for the oral tablet composition of the present invention

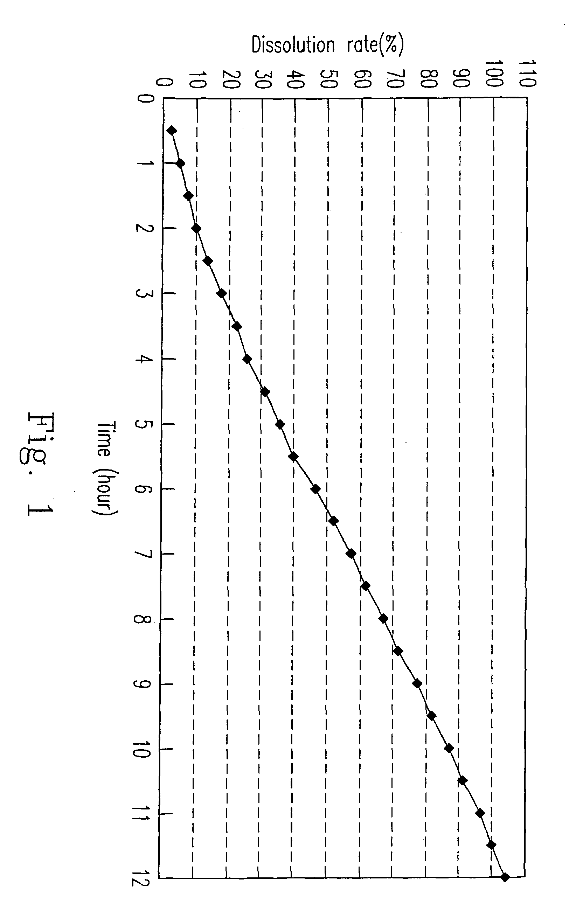

The tablet as prepared by Example 1 wherein micronized Gliclazide is as an active ingredient for the oral tablet is conducted to a dissolution test at 37°C by a USP type I Basket apparatus at 100 rpm in 900 ml of purified water, the testing result is shown in Table 1 and Fig. 1.

**Table 1**

| Time (hour) | Dissolution rate (%) |
|---|---|
| 0.5 | 2.0 |
| 1.0 | 4.0 |
| 1.5 | 6.2 |
| 2.0 | 9.5 |
| 2.5 | 12.9 |
| 3.0 | 16.6 |
| 3.5 | 21.2 |
| 4.0 | 25.8 |
| 4.5 | 30.6 |
| 5.0 | 35.6 |
| 5.5 | 40.8 |
| 6.0 | 46.2 |
| 6.5 | 51.5 |
| 7.0 | 56.6 |
| 7.5 | 61.4 |
| 8.0 | 66.5 |
| 8.5 | 71.5 |
| 9.0 | 76.0 |
| 9.5 | 80.6 |
| 10.0 | 85.2 |
| 10.5 | 89.3 |
| 11.0 | 93.0 |
| 11.5 | 96.5 |
| 12.0 | 100.1 |

From the result as shown above, about 5~15% of Gliclazide is released after dissolution for 2 hours, about 15~35% of Gliclazide is released after dissolution for 4 hours, about 50~80% of Gliclazide is released after dissolution for 8 hours, and about ≥85% of Gliclazide is released after dissolution for 12 hours.

### Dissolution Test

-in vitro testing comparison between an oral tablet composition for the present invention (test group) and an oral tablet composition comprising of an active ingredient for lowering the blood glucose level that has not been micronized (control froup).

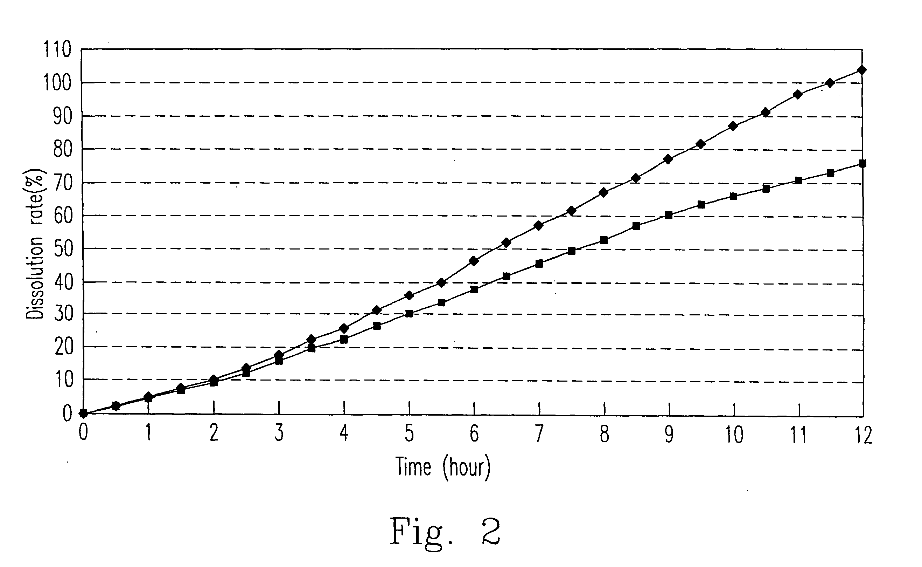

Micronized (Example 1) and non-micronized Gliclazide are used as an active ingredient for an oral tablet respectively and the resulted tablets are conducted to a dissolution test at 37°C by a USP type I Basket apparatus at 100 rpm in 900 ml of purified water, and the testing results are shown in Fig. 2. The diamond shape (◆) represents the tablet having a micronized active ingredient while the rectangular shape (■) represents the tablet having a non-micronized active ingredient; the results as shown above reveals that the tablet of the present invention has a better dissolution rate than the tablet having non-micronized Gliclazide after a longer dissolution time in the test, and the non-micronized form can not be completely released.

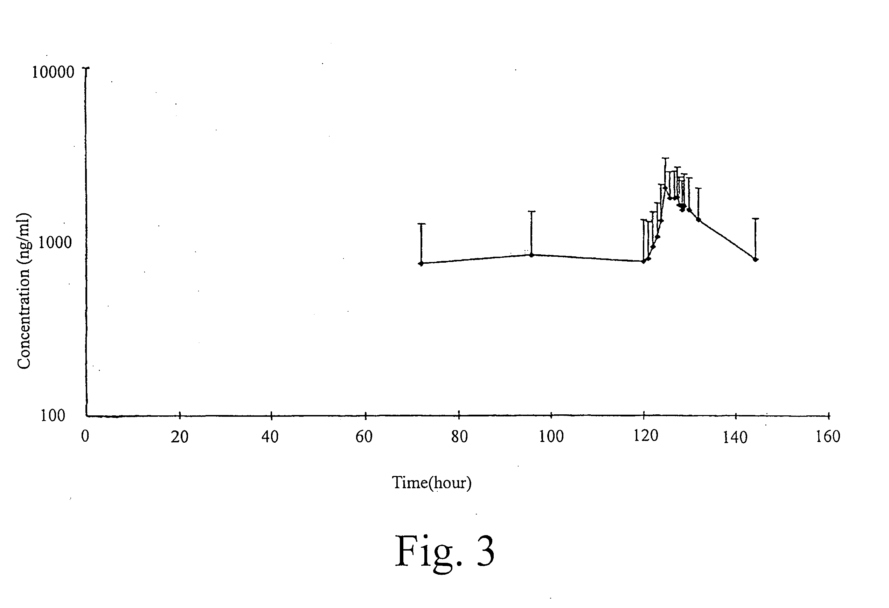

Meanwhile, please reference to Fig. 3 which shows the concentration change profile of the composition for a modified-release oral tablet in blood with a lapse of time, determined by the following procedures: a health candidate was selected according to the protocol requirement of a trial and was requested to take a drug for lowering blood glucose level as prepared by the present micronization process one time per day at regular interval and sequentially for six days, and blood withdrawn points were designed so as to monitor the drug concentration in the blood; the result shown in Fig. 3 reveals that the amount of the drug for lowering blood glucose level is sequential released to the blood with a lapse of long time, therefore the modified-release effect of the active ingredient for lowering the blood glucose level is achieved.

The documents as cited here are incorporated as references and each document can be as a part of disclosure for the present invention. The persons skilled in this art can easily make improvement from the examples without aberration of the applicability and principles of the present invention. Specific preferred examples are provided for the present invention, however, other improvement for the examples that is obvious to the persons skilled in the art is also within the scope of the present invention.

## Claims

1. A composition for a modified-release oral tablet, **characterized in that** comprising of:
a micronized active ingredient for lowering blood glucose level with a particle size distribution in the range of:
(1) at least 50% of the particles having a size ≤ 5 microns;
(2) at least 90% of the particles having a size ≤15 microns; and
(3) at least 95% of the particles having a size ≤25 microns; and
a modified-release agent for controlling the release of the active ingredient.

2. The composition for a modified-release oral tablet as claimed in claim 1, wherein the micronized active ingredient for lowering blood glucose level is selected from the group consisting of sulfonylureas, Gliclazide, Glipizide, Glibenclamide, Gliquidonem, Glimepiride and the combination thereof.

3. The composition for a modified-release oral tablet as claimed in claim 1, wherein the modified-release agent is selected from the group consisting of hydroxypropylmethyl cellulose, methyl cellulose, polyvinylpyrrolidone, hydroxypropyl cellulose, vinylacetate copolymers, polyethylene oxides and the combination thereof.

4. The composition for a modified-release oral tablet as claimed in claim 1, wherein the modified-release agent is 10%-40% by weight of the tablet composition.

5. The composition for a modified-release oral tablet as claimed in claim 1, wherein Gliclazide as a micronized active ingredient for lowering blood glucose level is with a weight between 30mg and 60mg.

6. The composition for a modified-release oral tablet as claimed in claim 5, wherein the micronized active ingredient for lowering blood glucose level having the following dissolution profile:
about 5~15% of the micronized active ingredient is released after dissolution for 2 hours;
about 15~35% of the micronized active ingredient is released after dissolution for 4 hours;
about 50~80% of the micronized active ingredient is released after dissolution for 8 hours; and
about ≥85% of the micronized active ingredient is released after dissolution for 12 hours.

7. The composition for a modified-release oral tablet as claimed in claim 5, wherein the composition is administered orally to a subject one time per day and 1-2 tablets per time.

8. The composition for a modified-release oral tablet as claimed in claim 5, wherein the composition comprises of micronized Gliclazide, hydroxypropylmethyl cellulose, polyvinylpyrrolidone, lactose, SiO2, polysorbate 80, magnesium stearate and talc.

9. The composition for a modified-release oral tablet as claimed in claim 3, wherein the viscosity of the modified-release agent is from 4,000 cps to 100,000 cps.

10. A method for preparation of a composition for a modified-release oral tablet, comprising of:
an active ingredient for lowering blood glucose level is micronized to a particle size distribution within the range of:
(1) at least 50% of the particles having a size ≤ 5 microns;
(2) at least 90% of the particles having a size ≤15 microns; and
(3) at least 95% of the particles having a size ≤25 microns; and
the said micronized active ingredient for lowering blood glucose level is mixed with a modified-release agent, subject to granulation, and then dried and trimmed; and
the resulting granulate is tabletted by the addition of an excipient, and a tablet with a hardness from 4 to 8 Kg is prepared.
